# EUROPEAN PATENT SPECIFICATION

(11) **EP 0 492 426 B1**
(45) Date of publication and mention of the grant of the patent: **20.09.1995**
(21) Application number: 91121755.2
(22) Date of filing: 19.12.1991
(51) Int. Cl.: C12N 1/20, C12P 19/18

(54) **Klebsiella oxytoca No. 19-1 and a process for producing alpha-cyclodextrin**
Klebsiella Oxytoca Nr. 19-1 und Verfahren zur Herstellung von Alpha-Cyclodextrin
Klebsiella oxytoca No. 19-1 et procédé pour la préparation de alpha-cyclodextrine

(30) Priority: 20.12.1990 KR 9021177
(43) Date of publication of application: 01.07.1992
(73) Proprietor: LOTTE CONFECTIONERY CO., Ltd., Seoul 150-104 (KR)
(72) Inventor: Jang-Youn, Choi, Bucheon-shi, Kyuonggi-Do (KR); Jae-Ho, Lee, Songpa-ku, Seoul (KR); Kee-Hyun, Choi, Sungdong-ku, Seoul (KR); Ik-Boo, Kwon, Sudaemoon-ku, Seoul (KR)
(74) Representative: Weber, Dieter, Dr.

(56) References cited:
- WO-A-89/01043
- Patent Abstracts of Japan, vol. 13, no. 549 (C-662) (3897) 7 December 1989 & JP-A-1 225 493 (NAKANO VINEGAR CO LTD) 8 September 1989

## Description

### BACKGROUND OF THE INVENTION

The present invention relates to a new strain of Klebsiella oxytoca No. 19-1 capable of producing α-cyclodextrin exclusively from starch, and to a process for producing α-cyclodextrin from starch with cyclodextrin glucanotransferase produced by the strain.

Cyclodextrins are cyclic oligosaccharides composed of glucose units and have known as host molecules which have torus in the molecule capable of forming inclusion complexes with various kinds of organic compounds, mainly with hydrophobic compounds.

Thus,cyclodextrins have been widely used in the fields of foods, chemicals, pharmaceuticals and agricultural chemistry and so on, in view of the following advantages:
- Modification of physical and chemical properties,for example,stabilization of volatile materials, protection against oxidation and UV degradation, increment in solubility of water-insoluble materials, shifting of colors, and deodorization.
- Emulsification of fats and oils.
- Acceleration and control of reaction, and improvement of yield.

Cyclodextrins are composed of 6,7 or 8 glucose residues which are bound by α-1,4-glucosidic linkage, and called α-, β- or γ-cyclodextrin depending on the number of carbon residue, 6,7 or 8, respectvely. The existence of branched cyclodextrins connected by α-1,6-glucosidic linkage has also been reported and the property of them is quite different from each other.

Extensive application of α-cylcodextrin among them, is expected in the food and medical industries, since α-cyclodextrin has higher solubility in water than β-cyclodextrin and is hardly digested by α-amylase of saliva and intestine, thereby classified as a non-metabolized dietary fiber which is not harmful to human body and digested by some Bacteroides when adsorbed into the body.

In conventional processes for producing α-cyclodextrin, starch as a substrate is reacted with an enzyme preparation selected from culture medium or various steps of purification which is obtained by cultivating microorganisms capable of producing cyclodextrin glucanotransferase, and α-cyclodextrin is then obtained from reaction mixtures by treatment of organic solvents or complicated processes, for example, gel filtration.

Up to now, various strains producing cyclodextrin have been public, particularly, there are typical strains producing α-cyclodextrin such as Bacillus macerans, Bacillus stearothermophilus and Klebsiella pneumoniae.

However, since the strains known as microorganisms producing α-cyclodextrin do not produce only α-cyclodextrin but produce simultaneously another types of cyclodextrins, it is still required to complicated purification process in preparation of α-cyclodextrin in high degree of purity.

Meanwhile, it was reported that the production of α-cyclodextrin was also increased by the presence of water-immiscible organic solvent like n-decyl alcohol and detergent like sodium dodecyl sulfate in the reaction mixture.

However, the use of toxic solvents or detergents seems to be undesirable since α-cyclodextrin produced using these are prohibited in food processing, and consequently it has confronted a problem requiring more complicated purification steps to remove the added chemical compounds.

Recently, Japanese Patent Laid-opened No. 89-225493 discloses a a method of producing α-cyclodextrin from Klebsiella pneumoniae subsp. pneumoniae α-CD, without any treatment of toxic solvents or detergents to promote the production of α-cyclodextrin. However, this method is still required to improve the process since there are some disadvantages in this method in point of concomitant production of large amounts of β-cyclodextrin, low production yield of α-cyclodextrin(10g/L),and prolonged reaction time of 3days, although this method does not produce branched cyclodextrins.

### SUMMARY OF INVENTION

The present invention aims to provide a new strain producing α-cyclodextrin exclusively from starch and also to provide process for producing α-cyclodextrin with cyclodextrin glucanotransferase produced by the strain.

### DETAILED DESCRIPTION OF THE PREFERRED EMBODIMENT

The present invention relates to a new strain, Klebsiella oxytoca No. 19-1 having the ability to produce α-cyclodextrin with a very high selectivity from starch.

And, the present invention includes a process producing α-cyclodextrin exclusively by reacting starch as a substrate with an enzyme preparation selected from cultured medium or various steps purification which is obtained by cultivating Klebsiella oxytoca No. 19-1(KCCM 10002) capable of producing cyclodextrin glucanotransferase.

It has been discovered by the present inventors that a newly isolated strain from soil and belonging to genus Klebsiella produces a similar enzyme to known one produced by the microorganisms belonging to genus Bacillus and Klebsiella.

It has been further discovered that the enzyme produced by a newly isolated strain from soil and belonging to genus Klebsiella has ability to produce α-cyclodextrin exclusively from starch.

It has been furthermore found that industrial production of the α-cyclodextrin can be advantageously accomplished by use of the enzyme since the enzyme produces α-cyclodextrin in very high proportion from starch, thereby simplify the manufacturing processes of α-cyclodextrin without an additional gel filtration process or treatment of organic solvent.

The microorganism according to the present invention belongs to genus Klebsiella and is identified as Klebsiella oxytoca.

This strain was named Klebsiella oxytoca No. 19-1 and was deposited with Korean Cultured Center and Microorganism on November 23,1990 as the deposit number KCCM 10002.

Taxonomical study of the strain was performed according to Bergey's Manual of Systematic Bacteriology and API 20E Kit(France).

Taxonomical characteristics of Klebsiella oxytoca No. 19-1 is as follows:
1. Morphological characteristics
   - Vegetable cell: : short-rod, 0.3~0.1µm × 0.6~3µm
   - Motility: : non-motile
   - Spore: : not formed
   - Gram staining: : negative
2. Cultural characteristics
   - Nutrient agar plate: : good growth, some protuberance and smoothness,dampness,having colony of lemon yellow.
   - Nutrient agar slant: : good growth, growing on the whole of slant.
   - MacConkey agar plate: : good growth,having colony of red pink color.
3. Physiological characteristics
   - pH for growth: : 4 ∼ 9
   - Temperature for growth: : 10∼35°C
   - Behavior to oxygen: : facultative anaerobic
   - Hydrolysis of starch: : positive
   - Hydrolysis of casein: : negative
   - Hydrolysis of carboxy methyl cellulose: : negative
   - Hydrolysis of pullulan: : positive
   - Liquefaction of gelatin: : negative
   - Citrates utilization: : positive
   - V.P. test: : positive
   - Indole production: : positive
   - H₂S production: : positive
   - M.R. test: : negative
   - Catalase: : positive
   - Oxidase: : negative
   - Urease: : positive
   - α-Galactosidase: : positive
   - Reduction of nitrate: : positive
   - Production of pigments: : negative
   - Tryptophane desaminase: : negative
   - Lysine decarboxylase: : positive
   - Ornithine decarboxylase: : negative
   - Arginine dihydrolase: : negative
   - Fecal coliform test: : negative
   [Acid production from various sugars ]
   - Glucose: : positive
   - Mannitol: : positive
   - Inositol: : positive
   - Sorbitol: : positive
   - Rhamnose: : positive
   - Sucorse: : positive
   - Melibiose: : positive
   - Amygdalin: : positive
   - Arabinose: : positive
In accordance with this invention, the process for producing α-cyclodextrin by using Klebsiella oxytoca No. 19-1 isolated from soil as the above mentioned is as follows.

The new strain is inoculated in a composited or natural medium and cultivated with shaking.

The medium should consist of starch or amylopectin as a carbon source, and in addition to that, a nitrogen source and inorganic salts may be used without limit. The strain is cultivated under the aerobic condition in pH adjusted to about 7, and then the culture temperature of 30∼40°C is prefered.

Cyclodextrin glucanotransferase from Klebsiella oxytoca No. 19-1 reached its maximum activity after 9 hour cultivation and further increase of activity was not observed after that time.

In order to produce α-cyclodextrin, the enzyme being in the supernatant of culture broth may be used in reaction with starch without purification but it is preferable to use the enzyme purified partially by general method.

Therefore, the present invention provides the efficient process for producing α-cyclodextrin without any aid of toxic solvents or detergents, and without complicated process like gel filtration to separate α-cyclodextrin from β- or γ-cyclodextrin in the reaction mixture, owing to the characteristics of the cyclodextrin glucanotransferase produced by Klebsiella oxytoca No. 19-1, when starch as a substrate was reacted with an enzyme preparation from cultured medium or partial purification of it under the conditions of pH 7 and 20-55°C .

Following are the examples to illustrate the present invention in in further detail but not to limit the scope of the invention.

### Example 1.

Klebsiella oxytoca No. 19-1 was cultivated aerobically in IL of the medium containing 1% soluble starch, 1% polypeptone, 0.1% K₂HPO₄, 0.02% MgSO₄; 37 °C. pH 7, 0.5vvm, 100rpm, 9hr.

After the removal of cells by centrifugation(6000×g, 5 min),supernatant was treated with three volumes of ethanol and maintained at 4°C for overnight.

The formed precipitate was collected by centrifugation(6000×g, 10 min, 4°C), suspended in 50mM phosphate buffer(pH 6),and dialyzed against same buffer for overnight.

Crude enzyme used in this invention was then obtained by lyophilization of the suspension.

The activity of cyclodextrin glucanotransferase was measured according to the method of Lejeune, A. et al. (Analytical Biochemistry, 181, p 6-11, 1989). 1ml of approximately diluted enzyme solution was incubated with 0.6ml of 5%(w/v) soluble starch,0.105ml of 1mM methyl orange and 1.295ml of 50mM phosphate buffer(pH 6) at 37°C for 10 min.

The reaction was ceased by addition of 0.150ml of 6N HCl and maintained at 15°C on a water bath for 30 min.

By determination of optical density at 507nm, the activity of the enzyme could be calculated with the prepared standard curve.

One unit of the enzyme activity was defined as the amount of enzyme which produces 1µmole of α-cyclodextrin per minute under the given conditions.

### Example 2.

10%(w/v) soluble starch solutions was prepared in 1 liter of 50mM phosphate buffer(pH 7) and 1mM CaCl₂.2H₂O and solubilized by autoclave for 5 min.

After cooling the solution, 0.5g of crude enzyme(1700 units) prepared by the above example 1 was added to the solution.

The reaction mixture was then incubated at 40 °C for 20hr with stirring and boiled on a water bath for 5 min to inactivate the enzyme.

Profile and content of cyclodextrins in the reaction mixture were determined by HPLC under the following conditions: Carbohydrate column (Waters Co., USA), RI detector, acetonitrile/water(65/35), flow rate 2.0 ml/min.

Before the analysis, the reaction mixture was treated with equal volume of eluent and filtered through a membrane(0.45 µm) to remove remaining substrate.

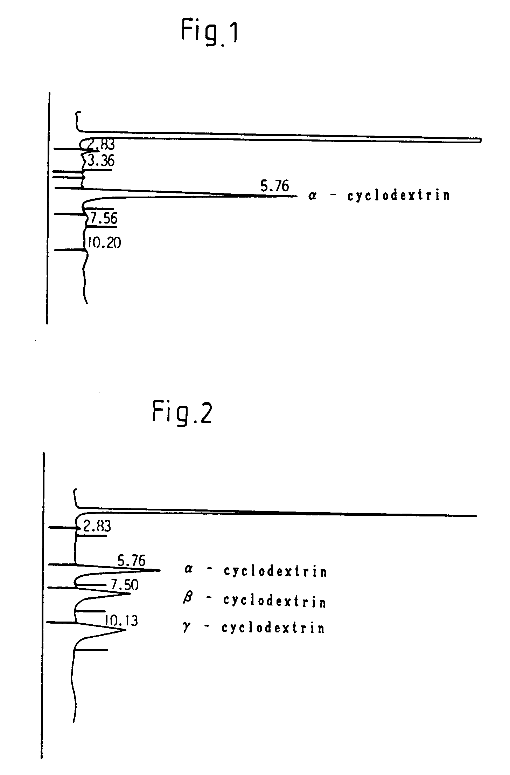

As shown in Fig 1, high amount of α-cyclodextrin was only detected and other cyclodextrins were not obsered.

The estimated concentration of α-cyclodextrin was 15g per 100g of starch when compared with standard cyclodextrins.

This result means that high amount of α-cyclodextrin is produced with a very high selectivity in a short reaction time without any aid of toxic solvent or detergent to increase the ratio of α-cyclodextrin in reaction mixtures.

Therefore, highly purified α-cyclodextrin product was easily prepared by concentration and drying processes from α-cyclodextrin in the reaction mixture and consequently, it was possible to eliminate the complicated process like gel filtration in manufacturing processes of α-cyclodextrin.

### BRIEF DESCRIPTION OF THE DRAWINGS

- Fig. 1: is a result of HPLC analysis for cyclodextrin reactant obtained after reaction for 20 hours according to example of this invention.
- Fig. 2: is a result of HPLC analysis for the known standard α -, β -,and τ -cyclodextrin produced on a commercial basis.

## Claims

1. Klebsiella oxytoca No. 19-1(Deposit No ; KCCM 10002) having the ability to digest starch and to produce α-cyclodextrin with a very high selectivity from starch.

2. A process for producing α-cyclodextrin exclusively by reacting starch as a substrate with an enzyme preparation selected from cultured medium which is obtained by cultivating microorganism, Klebsiella oxytoca No. 19-1 (Deposit No; KCCM 10002) capable of producing cyclodextrin glucanotransferase.

## Patentansprüche

1. Klebsiella oxytoca Nr. 19-1 (Hinterlegungs-Nr. KCCM 10002) mit der Fähigkeit, Stärke zu verdauen und mit sehr hoher Selektivität aus Stärke α-Cyclodextrin herzustellen.

2. Verfahren zur Herstellung von α-Cyclodextrin, ausschließlich durch Reaktion von Stärke als Substrat mit einer Enzympräparation, die von Kulturmedium abgetrennt wurde, welches durch Züchtung des Mikroorganismus Klebsiella oxytoca Nr. 19-1 (Hinterlegungs-Nr. KCCM 10002), der in der Lage ist, Cyclodextrin-Glucanotransferase zu produzieren, erhalten wird.

## Revendications

1. Klebsiella oxytoca N° 19-1 (N° de dépôt; KCCM 10002) ayant la capacité de digérer l'amidon et de produire de l'α-cyclodextrine avec une très grande sélectivité à partir de l'amidon.

2. Procédé de production d'α-cyclodextrine exclusivement en faisant réagir l'amidon comme substrat avec une préparation enzymatique choisie à partir de milieu de culture, qui est obtenue en cultivant des microorganismes, Klebsiella oxytoca N° 19-1 (N° de dépôt; KCCM 10002), capables de produire la cyclodextrine glucanotransférase.
